# EUROPÄISCHE PATENTSCHRIFT

(11) **EP 2 046 135 B1**
(45) Veröffentlichungstag und Bekanntmachung des Hinweises auf die Patenterteilung: **15.09.2010**
(21) Anmeldenummer: 07786350.4
(22) Anmeldetag: 26.07.2007
(51) Int. Cl.: A23L 1/30, A23L 1/305, A23L 2/52, A23L 2/66, A61K 38/01, A61K 38/17, A23G 1/44, A23G 1/46, A61K 31/353, A61P 1/12

(54) **POLYPHENOL-PEPTID-ZUSAMMENSETZUNG UND IHRE VERWENDUNG ZUR UNTERSTÜTZUNG DER DARMGESUNDHEIT**
POLYPHENOL/PEPTIDE COMPOSITION AND USE THEREOF FOR PROMOTING GASTRIC HEALTH
COMPOSITION A BASE DE POLYPHENOL ET DE PEPTIDE QUI SOUTIENT LA SANTE INTESTINALE

(30) Priorität: 28.07.2006 DE 102006034924; 08.11.2006 DE 102006052915
(43) Veröffentlichungstag der Anmeldung: 15.04.2009
(73) Patentinhaber: Humana Milchunion EG, 48351 Everswinkel (DE)
(72) Erfinder: SELL, Marco, 25866 Mildstedt (DE); SAWATZKI, Günther, 35516 Münzenberg (DE)
(74) Vertreter: Tappe, Hartmut
(86) Internationale Anmeldenummer: PCT/EP2007/006631
(87) Internationale Veröffentlichungsnummer: WO 2008/012089

(56) Entgegenhaltungen:
- EP-A- 0 291 265
- EP-A- 0 745 332
- WO-A-02/052954
- DE-A1- 10 016 771
- DE-A1- 19 627 344
- US-A1- 2002 064 584
- US-A1- 2003 008 810
- SCHUIER, M., SIES, H., ILLEK, B., FISCHER, H.: "Cocoa related flavonoids inhibit CFTR mediated chloride transport across T84 human colon epithelia" J NUTRITION, Bd. 135, 2005, Seiten 2320-2325, XP002463651 in der Anmeldung erwähnt
- NAKAJIMA, K., TAMURA, N., KOBAYASHI-HATTORI K. ET AL.: "Prevention of intestinal infection by glycomacropeptide" BIOSCI BIOTECHNOL BIOCHEM, Bd. 69, Nr. 12, 2005, Seiten 2294-2301, XP002463652 in der Anmeldung erwähnt
- ISHIHARA N ET AL: "GREEN TEA EXTRACT AS A REMEDY FOR DIARRHEA IN FARM-RAISED CALVES" CHEMISTRY AND APPLICATIONS OF GREEN TEA, 1997, Seiten 137-144, XP008032065

## Beschreibung

Die vorliegende Erfindung betrifft eine Zusammensetzung, die eine erste Komponente aus der Gruppe der Polyphenole und eine zweite Komponente aus der Gruppe der Milch-Peptide enthält. Des Weiteren betrifft die Erfindung ein Verfahren zur Herstellung der Zusammensetzung und die Verwendung der Zusammensetzung zur Herstellung von Produkten, die entweder als Arzneimittel oder als diätetisches Lebensmittel Verwendung finden.

Als Polyphenole werden reduzierende Substanzen bezeichnet, die in ihrem Kohlenstoff-Skelett eine oder mehrere phenolische Gruppen, wie zum Beispiel Diphenole, Phenolsäuren, Flavonole und Lignane enthalten. Dazu gehören als Untergruppe die so genannten Flavonoide, die wiederum in die Procyanidine und Catechine eingeteilt werden (A. Scalbert and G. Williamson, Dietary Intake and Bioavailability of Polyphenols, J. Nutr. 130: 2073S-2085S (2000)). Natürliche Quellen für Polyphenole sind schwarzer oder grüner Tee, verschiedene Früchte (Äpfel und Weintrauben) und insbesondere Kakao. Schon bei den Indianern Mittelamerikas wurde Kakao (hier als Quelle für Polyphenole) seit einigen Jahrhunderten (seit dem 15. Jahrhundert) zu Heilzwecken eingesetzt (T.L. Dillinger, P. Barriga, S. Escarcega, M. Jimenez, D.S. Lowe and L.E. Grivetti, Food of the Gods: Cure for Humanity? A cultural History of the Medicinal and Ritual Use of Chocolate, J. Nutr. 130: 2057S - 2072S (2000)). Eine Anwendung war dabei die Behandlung von Durchfallerkrankungen. Erst in den letzten Jahren hat es sich herausgestellt, dass zu den wirksamsten Substanzen im Kakao die so genannten Polyphenole zählen. Intensive Erforschung der Einzelsubstanzen ist von verschiedenen Forschungsgruppen in den letzten Jahren betrieben worden und insbesondere die so genannten Flavonole und davon wiederum die so genannten Catechine sind intensiv auf ihre biologische Wirkung untersucht worden. Erst in den letzten Jahren wurden die Catechine, insbesondere die Substanz Epicatechin, intensiv auf ihre biologischen Wirkungen untersucht. Seit etwa einem Jahr ist der Wirkungsmechanismus von Epicatechin auf die Darmzellwand genauer untersucht und entschlüsselt worden. Dabei stellte sich heraus, dass Epicatechin direkt auf ein Transportsystem in den Darmzellen einwirkt, das den Wassertransport in das Darmlumen bewirkt. Hauptproblem bei der Behandlung von Durchfallerkrankungen ist der enorme Wasserverlust, der durch den Ausstrom von Wasser aus dem Körper in den Darm verursacht wird. Hierbei kommt Transportsystemen der Darmzellen eine Schlüsselstellung zu. Hier sei der wichtige Chloridkanal (CFTR= Cystic Fibrosis Transmembrane Conductance Regulator) genannt, der für den Wassertransport in das Darmlumen wichtig ist. Für Epicatechin wurde nun gefunden, dass es genau dieses Transportsystem für Wasser unterdrückt (M. Schuier, H. Sies, B. Illek and H. Fischer, Cocoa-related Flavonoids inhibit CFTR-mediated Chloride Transport across T84 Human colon epithelia, J. Nutr. 135: 2320-2325 (2005)). In diesem Zusammenhang wurde auch darauf verwiesen, dass die wichtigste Quelle für Epicatechin in unserer Nahrung Kakao und Schokolade ist.

Es ist zudem in der Patentliteratur bereits beschrieben, dass man Flavonoide für gesundheitsfördernde Produkte ( DE 196 27 344 A1, US 6,599,553 B2) und auch zur Behandlung von Darmerkrankungen einsetzen kann (DE 299 08 634 U1, DE 100 16 771 C2, US 2003/0113290 A1) jedoch wurde die Wirkung nie mit einer Peptidkomponente gezielt verstärkt.

Eine ganz besondere Stellung nehmen die Polyphenole des Kakao ein, da ebenso auch ein antibakterieller Effekt von Kakao auf enterohämorrhagische Escherichia coli (E. coli) Bakterien gefunden wurde. (T. Takahashi, H. Taguchi, H. Yamaguchi, T. Osaki, S. Sato, M. Kamei, S. Hashizume and S. Kamiya, Antibacterial effects of cacao mass on enterohemorrhagic Escherichia coli O157:H7 Kansenshogaku Zasshi 73:694-701 (1999)).

Peptide aus der Milch sind in den letzten Jahren, insbesondere wegen ihrer biologischen Funktionen und Wirkungen, in das Interesse der Forschung gelangt. Die meisten Peptide aus der Milch entstehen durch Hydrolyse aus größeren Milchproteinen. Bei der Herstellung von Käse wird durch die hydrolytische Aktivität des so genannten Labenzyms aus dem Casein das so genannte Glycomacropeptid abgespalten. Dieses Peptid entsteht bei der Käsegewinnung in sehr großen Mengen und befindet sich in der Molke, die bei der Käsegewinnung anfällt. In den letzten Jahren haben verschiedene Forschungsgruppen untersucht welche biologischen Funktionen dieses Glycomacropeptid aufweist. Interessanterweise konnte auch eine antimikrobielle Wirkung nachgewiesen werden (K. Nakajima, N. Tamura, K. Kobayashi-Hattor, T. Yoshida, Y. Hara-Kudo, M. Ikedo, Y. Sugata-Konishi and M. Hattori, Prevention of Intestinal Infection by Glycomacropeptide, Biosci. Biotechnol. Biochem. 69: 2294-2301 (2005)). So wurde eine wachstumshemmende Wirkung gerade bei den Bakterien gefunden, die bei Diarrhöen häufig auftreten, nämlich E. coli (insbesondere der Stamm EHEC 0157). Erwähnenswert ist in diesem Zusammenhang noch, dass die Glycosylierung bei dieser Funktion des Glycomacropeptids eine gewisse Rolle spielt. So wird der Sialinsäure an dem Glycomacropeptid eine Funktion zugeschrieben.

Die US 2002/0064584 A1 offenbart ein Kakaoprodukt mit einem erhöhten Gehalt an Kakao-Polyphenolen u.a. Epicatechin. Diesem Produkt können auch Milchfeststoffe, die Milch- und Molkenpeptide sowie Glycomacropeptid enthalten, zugefügt werden.

Die US 2003/0008810 A1 beschreibt die Verwendung einer Zusammensetzung, welche Glycomacropeptid als Wirkstoff enthält, zur Verringerung der Kalorienaufnahme. Dieser ggf. auch flüssigen Zusammensetzung kann Tee hinzugefügt werden, der Polyphenole, insbesondere auch Epicatechin, enthält.

Die WO 02/052954 A2 offenbart die Verwendung von Soja-Isoflavonen, die zur Gruppe der Polyphenole gehören, in Kombination mit Glycomacropeptid zur Prävention oder Behandlung von Knochenerkrankungen.

Die EP 0 745 332 A1 beschreibt eine Zusammensetzung für verschiedene therapeutische Zwecke, welche partiell hydrolisierte und denaturierte Molkenproteine und ein Allium-Extrakt mit hohem Flavonoidgehalt enthält.

Obwohl es, wie vorstehend ausgeführt, schon bekannt ist, dass verschiedene Komponenten aus Nahrungsmitteln auf Diarrhöe-auslösende Bakterien und Viren eine wachstumshemmende Wirkung zeigen und auch ansonsten für die Darmgesundheit förderlich sind, besteht immer noch der Bedarf nach einem einfach verabreichbaren, kostengünstigen und nebenwirkungsarmen Mittel zur Förderung der Darmgesundheit, das auch Diarrhöen wirksam bekämpfen kann. Die Aufgabe der vorliegenden Erfindung besteht daher darin ein solches Mittel bereitzustellen.

Die vorliegende Erfindung betrifft daher eine Polyphenol-Peptid-Zusammensetzung, die eine Komponente aus der Gruppe der Polyphenole enthält und eine zweite Komponente aus der Gruppe der Milch-Peptide enthält, wie beansprucht.

Als Polyphenol-Komponente wird erfindungsgemäß ein Epicatechin aus verschiedenen Nahrungsquellen, insbesondere aus Kakao, verwendet.

Erfindungsgemäß wird als Peptid-Komponente um das Glycomacropeptid verwendet.

Überraschenderweise hat sich nun herausgestellt, dass die Wirkung auf die Diarrhöe bei einer Kombination aus Polyphenolen (Epicatechin) und einem Peptid aus der Milch (Glycomacropeptid) wesentlich stärker ausgeprägt ist, als die Wirkung der Einzelkomponenten. So konnte in Untersuchungen der Erfinder festgestellt werden, dass bei Gabe einer konventionellen Glukose-Elektrolyt-Lösung (A), bei einem Zusatz nur von Polyphenolen (B) oder nur von Milchpeptiden (C) verglichen mit der erfindungsgemäßen Zusammensetzung in Form einer Kombination von Polyphenolen und Milchpeptiden (D) eine deutliche Verkürzung der Dauer der Diarrhöe (A = 66 Stunden, B = 47 Stunden, C = 55 Stunden, D = 30 Stunden) nachzuweisen war. Somit steht in Form dieser Polyphenol-Peptid-Zusammensetzung eine biologisch hochwirksame Form eines Stoffgemisches zur Behandlung von Diarrhöen zur Verfügung. Dabei kann diese Komponente sowohl therapeutisch in Arzneimitteln wie auch unterstützend in Form von diätetischen Lebensmitteln eingesetzt werden.

Grundsätzlich kann die Polyphenol-Peptid-Zusammensetzung zur Herstellung von Produkten in flüssiger, fester, pulverförmiger, kompaktierter oder beliebiger anderer Aggregatszustände eingesetzt werden. Mit dem Aggregatzustand des Produkts ergibt sich auch die Applikationsform des Produkts. Bevorzugt ist die orale oder rektale Applikation. Ebenso möglich ist aber auch die intragastrale (direkt in den Magen), die nasogastrale (über eine Sonde durch die Nase in den Magen), die parenterale (direkt in den Körper unter Umgehung des Magendarmtraktes) oder die intravenöse (direkt in eine Blutvene) Applikation.

Die erfindungsgemäße Zusammensetzung kann des Weiteren die in der pharmazeutischen Industrie oder Nahrungsmittelindustrie üblichen Hilfs-, Füll- und Zusatzstoffe enthalten. Es können dabei des Weiteren Kohlenhydrate, Proteine, Mineralstoffe, Salze und/oder Vitamine enthalten sein.

Beispielhaft werden nachfolgend Produkte beschrieben, die im diätetischen Nahrungsmittelbereich eingesetzt werden. Es können aber auch Produkte hergestellt werden, die dem Bereich der pharmazeutischen Produkte zuzuordnen sind. Erfindungsgemäß kann die Zusammensetzung in pharmazeutischen Produkten zur Behandlung von Diarrhöen eingesetzt werden sowie in diätetischen Produkten zur Unterstützung der Darmgesundheit und bei Maßnahmen zur Prophylaxe und Nachbehandlung von Diarrhöen.

Erfindungsgemäß haben sich folgende Tagesdosen für Erwachsene oder Kleinkinder/Säuglinge der gewünschten Inhaltsstoffe bewährt:

| Inhaltsstoff | Für Säuglinge | Für Kleinkinder | Für Erwachsene |
|---|---|---|---|
| Polyphenol: (Epicatechin) | 0,05 bis 30 mg, | 5 bis 70 mg, | 1 bis 300 mg, |
| | bevorzugt: | bevorzugt: | bevorzugt: |
| | 0,1 bis 25 mg, | 10 bis 50 mg, | 10 bis 100 mg, |
| | ganz bevorzugt: | ganz bevorzugt: | ganz bevorzugt: |
| | 5 bis 20 mg | 15 bis 40 mg | 15 bis 80 mg |
| Milch-Peptid (Glycomacropeptid) | 0,1 bis 8 g, | 0,1 bis 15 g, | 0,1 bis 30 g, |
| | bevorzugt: | bevorzugt: | bevorzugt: |
| | 0,2 bis 6 g, | 0,2 bis 12 g, | 0,5 bis 20 g, |
| | ganz bevorzugt: 0,5 bis 10 | ganz bevorzugt: | ganz bevorzugt: |
| | 0,5 bis 5 g | 0,5 bis 10 g | 1 bis 10 g |

Die Erfindung wird nachfolgend anhand von Beispielen, die als nicht beschränkend auszulegen sind, beschrieben.

### Beispiel 1: Elektrolyt, ein diätetisches Lebensmittel zur Behandlung von Durchfallerkrankungen; als Pulver

Zusammensetzung, geeignet für die Behandlung von Säuglingen:

| Inhaltsstoff | pro 100 g Pulver | pro Portion (250 ml) |
|---|---|---|
| Epicatechin in mg | 80 | 5 |
| Glycomacropeptid in mg | 8000 | 500 |
| Glucose in g | 40 | 2,5 |
| Maltodextrin in g | 2,9 | 0,2 |
| Natrium in mg | 5552 | 347 |
| Kalium in mg | 3120 | 195 |
| Chlorid in mg | 7040 | 440 |
| Citrat in mg | 7472 | 467 |

### Rezeptur:

| | |
|---|---|
| Glucose | 40,2 g |
| Kakaopulver | 25 g |
| GMP | 8 g |
| Natriumchlorid | 11,6 g |
| Maltodextrin | 2,9 g |
| Kaliumcitrat | 8,15 g |
| Natriumcitrat | 4,18 g |

### Herstellvorschrift:

- Komponenten einzeln einwiegen
- mischen
- abfüllen

### Beispiel 2: Elektrolyt, ein diätetisches Lebensmittel zur Behandlung von Durchfallerkrankungen, in flüssiger Form.

### Zusammensetzung, geeignet für Erwachsene:

| Inhaltsstoff | pro 100 ml | pro Portion (250 ml) |
|---|---|---|
| Epicatechin in mg | 10 | 25 |
| Glycomacropeptid in mg | 800 | 2000 |
| Glucose in g | 1,6 | 4 |
| Maltodextrin in g | 0,28 | 0,7 |
| Natrium in mg | 139 | 347 |
| Kalium in mg | 78 | 195 |
| Chlorid in mg | 176 | 440 |
| Citrat in mg | 187 | 467 |

### Rezeptur:

| | |
|---|---|
| entmineralisiertes Wasser | 94,2 g |
| Kakaopulver | 3,2 g |
| Glucose | 1,6 g |
| GMP (66 % Reinheit) | 1,2 g |
| Maltodextrin | 0,3 g |
| Natriumchlorid | 0,3 g |
| Kaliumcitrat | 0,2 g |

### Herstellvorschrift:

- Wasser im Mischbehälter vorlegen
- Pulverkomponenten einzeln in den Mischbehälter unter Rühren zugeben
- durchmischen bis homogene Verteilung der Komponenten erfolgt ist
- Abfüllung

### Beispiel 3: Heilnahrung flüssig

### Rezeptur:

| | | |
|---|---|---|
| entmineralisiertes Wasser | 54,6 | g |
| entrahmte Milch | 25 | g |
| Bananenpüree | 11 | g |
| Glucosesirup | 3,4 | g |
| Kakaopulver | 3,2 | g |
| Quellstärke | 2,5 | g |
| Pflanzliches Fett | 2,0 | g |
| GMP (66 % Reinheit) | 1,2 | g |
| Calciumcaseinat | 0,9 | g |
| Reismehl | 0,6 | g |
| Mineralstoffmischung (Kaliumcitrat, Calciumcarbonat, Natriumcitrat, Natriumcarbonat, Natriumchlorid, Magnesiumcarboant, Eisendiphosphat, Zinksulfat, Kupfersulfat, Kaliumjodat, Natriummolybdat, Natriumselenat, Chromchlorid) | 156,1 | mg |
| Emulgator: Sojalecithin | 30 | mg |
| Vitaminmischung (Vitamin C, Vitamin E, Niacin, Pantothenat, Vitamin B1, Vitamin B2, Vitamin B6, Vitamin A, Folsäure, Vitamin K, Biotin, Vitamin D3, Vitamin B12) | 22,5 | mg |

### Herstellvorschrift:

- Wasser und Magermilch im Mischbehälter vorlegen
- Bananenpüree zudosieren
- Pulverkomponenten einzeln in den Mischbehälter unter Rühren zugeben
- Fett zudosieren
- durchmischen bis homogene Verteilung der Komponenten erfolgt ist
- Homogenisierung
- Kühlung der Emulsion
- Abfüllung

### Beispiel 4: Heilnahrung als Pulver

### Rezeptur:

| | | |
|---|---|---|
| entrahmte Milch | 127 | g |
| Bananenpüree | 67,0 | g |
| Glucosesirup | 22,6 | g |
| Quellstärke | 12,2 | g |
| Pflanzliches Öl | 13,3 | g |
| Calciumcaseinat | 8,1 | g |
| Reismehl | 2,5 | g |
| Kakaopulver | 4,6 | g |
| Maltodextrine | 2,5 | g |
| entmineralisiertes Molkenpulver | 2,5 | g |
| GMP (66 % Reinheit) | 2,1 | g |
| Mineralstoffmischung (Kaliumcitrat, Natriumcitrat, Calziumcarbonat, Natriumchlorid, Magnesiumcarbonat, Eisendiphosphat, Zinkoxid, Kupfersulfat, Kaliumjodat, Natriummolybdat, Chromchlorid, Natriumselenat) | 1,6 | g |
| Vitaminmischung (Vitamin C, Vitamin E, Niacin, Pantothenat, Vitamin B1, Vitamin B2, Vitamin B6, Vitamin A, Folsäure, Vitamin K, Biotin, Vitamin D3, Vitamin B12) | 140 | mg |
| Emulgator: Sojalecithin | 360 | mg |

### Herstellvorschrift:

- Pulverkomponenten einzeln in den Mischbehälter unter Rühren zugeben
- durchmischen bis homogene Verteilung der Komponenten erfolgt ist
- Abfüllung

### Beispiel 5: Nachweis der Wirkung in einer Untersuchung

In einer Untersuchung bei Säuglingen und Kleinkindern im Alter von 6 bis 48 Monaten wurden folgende Elektrolyt-Lösungen eingesetzt:
1. eine konventionelle Glukose-Elektrolytlösung (analog zu Beispiel 2, aber ohne Polyphenole und Milchpeptide) = Gruppe A
2. eine Glukose-Elektrolytlösung (analog zu Beispiel 2, aber nur mit Polyphenolen) = Gruppe B
3. eine Glukose-Elektrolytlösung (analog zu Beispiel 2, aber nur mit Milchpeptiden) = Gruppe C
4. eine Glukose-Elektrolytlösung (Beispiel 2, komplett mit Polyphenolen und Milchpeptiden, erfindungsgemäße Zusammensetzung) = Gruppe D

Es wurden jeweils 50 ml der Lösungen pro kg Körpergewicht in 4 Stunden oral gegeben. Die Behandlung wurde so lange fortgesetzt, bis innerhalb von 24 Stunden nur noch 2 nichtwässrige Stühle abgesetzt wurden (= Ende der Diarrhöe). Dabei ergaben sich folgende Zeiten bis zum Ende der Krankheitszeichen:
Gruppe A = 66 Stunden,
Gruppe B = 47 Stunden,
Gruppe C = 55 Stunden,
Gruppe D = 30 Stunden.

Neben der deutlich kürzeren Krankheitsdauer gegenüber der zusatzlosen Elektrolyt-Lösung oder den Einzelzusätzen, zeigte die erfindungsgemäße Kombination auch eine deutlich bessere Gewichtszunahme innerhalb von 24 Stunden (+5,4 %), eine schnellere Abnahme der Azidosen (15 Stunden kürzer) und eine schnellere Abnahme der deutlich wässrigen Stühle, bereits nach 24 Stunden.

Einzig und allein in der Gruppe D (mit der erfindungsgemäßen Kombination) konnte im Stuhl bereits nach 24 Stunden eine deutliche Verringerung an pathogenen Bakterien (E. coli) und Viren (Rotaviren) nachgewiesen werden. Diese Verringerung war in den Gruppen A und B überhaupt nicht zu beobachten und in der Gruppe C vergleichsweise schwächer und deutlich später (48 Stunden) zu sehen.

Somit konnte eindrucksvoll der synergistische Effekt der erfindungsgemäßen Kombination gezeigt werden.

## Patentansprüche

1. Polyphenol-Peptid-Zusammensetzung enthaltend als Polyphenol-Komponente das Polyphenol Epicatechin in einer Menge von 0,1 bis 80 mg pro 100 g Produkt und als PeptidKomponente das Molkenpeptid Glycomacropeptid in einer Menge von 0,5 bis 10 g pro 100 g Produkt.

2. Zusammensetzung nach Anspruch 1, wobei des Weiteren Kohlenhydrate, Proteine, Mineralstoffe, Salze und/oder Vitamine enthalten sind.

3. Zusammensetzung nach Anspruch 1 oder 2, wobei sie gemischt mit anderen Komponenten in Form flüssiger, fester, pulverförmiger, kompaktierter oder beliebiger anderer Aggregatzustände für ein fertiges Produkt eingesetzt wird.

4. Verwendung einer Polyphenol-Peptid-Zusammensetzung enthaltend Epicatechin und Glycomacropeptid zur Herstellung von pharmazeutischen Produkten zur Behandlung von Diarrhöen.

5. Verwendung einer Polyphenol-Peptid-Zusammensetzung enthaltend Epicatechin aus Kakao und Glycomacropeptid zur Herstellung von diätetischen Produkten zur Unterstützung der Darmgesundheit und bei Maßnahmen zur Prophylaxe und Nachbehandlung von Diarrhöen.

6. Verwendung einer Zusammensetzung nach einem der Ansprüche 1 bis 3 zur Herstellung von pharmazeutischen Produkten zur Behandlung von Diarrhöen.

7. Verwendung einer Zusammensetzung nach einem der Ansprüche 1 bis 3 zur Herstellung von diätetischen Produkten zur Unterstützung der Darmgesundheit und bei Maßnahmen zur Prophylaxe und Nachbehandlung von Diarrhöen.

8. Verfahren zur Herstellung einer Zusammensetzung nach einem der Ansprüche 1 bis 3, wobei eine Polyphenol-Komponente mit einer Milchpeptid-Komponente sowie gegebenenfalls weiteren Hilfs-, Füll- und/oder Zusatzstoffen gemischt wird.

## Claims

1. A polyphenol/peptide composition comprising as polyphenol component the polyphenol epicatechin in an amount of 0.1 to 80 mg per 100 g of product and as peptide component the whey peptide glycomacropeptide in an amount of 0.5 to 10 g per 100 g of product.

2. A composition according to claim 1, said composition further comprising carbohydrates, proteins, mineral substances, salts and/or vitamins.

3. A composition according to claim 1 or claim 2, said composition mixed with other components being used in the form of liquid, solid, powdery, compacted or any other states of aggregation for a finished product.

4. The use of a polyphenol/peptide composition comprising epicatechin and glycomacropeptide for producing pharmaceutical products for treating diarrheas.

5. The use of a polyphenol/peptide composition comprising epicatechin from cacao and glycomacropeptide for producing dietetic products for promoting intestinal health and in measures for the prophylaxis and after-treatment of diarrheas.

6. The use of a composition according to any one of claims 1 to 3 for producing pharmaceutical products for treating diarrheas.

7. The use of a composition according to any one of claims 1 to 3 for producing dietetic products for promoting intestinal health and in measures for the prophylaxis and after-treatment of diarrheas.

8. A process for producing a composition according to any one of claims 1 to 3 wherein a polyphenol component is mixed with a lactic peptide component and optionally additional excipients, fillers and/or additives.

## Revendications

1. Composition de polyphénol/peptide contenant en tant que composant polyphénolique le polyphénol épicatéchine dans une quantité de 0,1 à 80 mg pour 100 g de produit et en tant que composant peptidique le peptide laitier glycomacropeptide dans une quantité de 0,5 à 10 g pour 100 g de produit.

2. Composition selon la revendication 1, contenant par ailleurs des hydrates de carbone, des protéines, des minéraux, des sels et/ou des vitamines.

3. Composition selon la revendication 1 ou 2, celle ci étant utilisée pour un produit fini en étant mélangée à d'autres composants sous forme d'agrégats à l'état liquide, solide, pulvérulent, compacté ou autre forme quelconque.

4. Utilisation d'une composition de polyphénol/peptide contenant de l'épicatéchine et un glycomacropeptide pour la production de produits pharmaceutiques destinés au traitement de diarrhées.

5. Utilisation d'une composition de polyphénol/peptide contenant de l'épicatéchine de cacao et un glycomacropeptide pour la production de produits diététiques pour favoriser la santé intestinale et pour des mesures de prophylaxie et de post-traitement de diarrhées.

6. Utilisation d'une composition selon l'une quelconque des revendications 1 à 3 pour la production de produits pharmaceutiques destinés au traitement de diarrhées.

7. Utilisation d'une composition selon l'une quelconque des revendications 1 à 3 pour la production de produits diététiques pour favoriser la santé intestinale et pour des mesures de prophylaxie et de post-traitement de diarrhées.

8. Procédé de fabrication d'une composition selon l'une quelconque des revendications 1 à 3, un composant polyphénolique étant mélangé avec un composant de peptide laitier, ainsi que le cas échéant avec d'autres produits consommables secondaires, matières de charge et/ ou adjuvants.
